# EUROPEAN PATENT SPECIFICATION

(11) **EP 2 095 802 B1**
(45) Date of publication and mention of the grant of the patent: **13.02.2013**
(21) Application number: 09250545.2
(22) Date of filing: 27.02.2009
(51) Int. Cl.: A61F 13/511, A61F 13/512

(54) **Topsheet for an absorbent article**
Oberblatt eines absorbierenden Artikels
Feuille supérieure d'article absorbant

(30) Priority: 29.02.2008 TW 97107029
(43) Date of publication of application: 02.09.2009
(73) Proprietor: Huang, Chen-Cheng, Taipey City (TW); Huang, Pao-Han, Taipei City (TW); Huang, Pao-Hao, Taipei City (TW)
(72) Inventor: Huang, Chen-Cheng, Taipey City (TW); Huang, Pao-Han, Taipei City (TW); Huang, Pao-Hao, Taipei City (TW)
(74) Representative: Schwerbrock, Florian

(56) References cited:
- JP-A- 2004 113 489
- US-A- 3 221 738
- US-A- 3 602 220
- US-A1- 2002 034 914
- US-A1- 2002 049 419
- US-A1- 2003 191 442
- US-A1- 2004 127 875
- US-A1- 2004 140 047
- US-A1- 2004 254 554
- US-A1- 2004 265 533
- US-B1- 6 362 391
- US-B1- 6 436 082

## Description

This invention relates to a topsheet for an absorbent article, more particularly to a topsheet including a base wall and a plurality of strip-like protrusions protruding from the base wall to form a plurality of fluid channels for spreading liquid in a desired direction.

U.S. Patent No. 6,461,339 discloses an absorbent article that includes a liquid pervious topsheet, a liquid impervious backsheet, an absorbent core disposed between the topsheet and the backsheet, and a non-woven inner layer disposed between the topsheet and the absorbent core. The topsheet has an outer layer of a non-woven fabric, and a perforated plastic film layer that is in contact with the non-woven inner layer. The outer layer may be embossed, and serves mainly for providing softness and to absorb moisture so that a sticky or sweaty feel is not experienced by the user.

The outer layer is formed with an opening extending from a front edge of the absorbent article to a rear edge of the absorbent article for passage of a liquid to be deposited directly onto the perforated plastic film layer so that the liquid can rapidly pass into the absorbent core through the perforated plastic film layer and the non-woven inner layer.

By virtue of the structures of apertures formed in the perforated plastic film layer, pores formed in the outer layer, and pores formed in the non-woven inner layer, liquid deposited on the outer layer can effectively pass through the apertures and the pores in a normal direction relative to the absorbent article and into the absorbent core, but cannot effectively flow in a lengthwise direction of the absorbent article. As a consequence, liquid flow from the outer layer into the absorbent core occurs only over a limited area of the absorbent article, i.e., a wet area where the liquid is deposited.

It has been proposed that the fibers used for forming the non-woven inner layer be oriented in a specific direction before they are entangled together so as to permit spreading of the liquid in the lengthwise direction and so as to increase the wet area over which the liquid flows through the outer layer, the perforated plastic film layer, and the non-woven inner layer and into the absorbent core. However, the non-woven inner layer thus formed is expensive.

Hence, there is a need for a topsheet that is cost effective and that can effectively cause spreading of liquid in the lengthwise direction.

Therefore, an object of the present invention is to provide a topsheet having a configuration that can effectively cause spreading of a liquid body and dispense with the aforementioned non-woven inner layer employed in the prior art.

US-B1-6 436 082 (MIZUTANI SATOSHI [JP] ET AL) 20 August 2002 (2002-08-20)
US 2004/254554 A1 (MAVINKURVE PRAMOD S [US] ET AL) 16 December 2004 (2004-12-16)
US-A-3 602 220 (BUNYAN JOHN) 31 August 1971 (1971-08-31) US2002/049419 A1 (MIZUTANI SATOSHI [JP] ET AL) 25 April 2002 (2002-04-25)
US 2004/265533 A1 (HOYING JODY LYNN [US] ET AL) 30 December 2004 (2004-12-30)
US 2004/140047 A1 (SATO KENICHI [JP] ET AL) 22 July 2004 (2004-07-22)
US-B1-6 362391 (MIZUTANI SATOSHI [JP] ET AL) 26 March 2002 (2002-03-26)
US 2003/191442 A1 (BEWICK-SONNTAG CHRISTOPHER PHI [US] ET AL) 9 October 2003 (2003-10-09)
JP 2004 113489 A1 (ASAHI KASEI FIBERS CORP)15 April 2004 (2004-04-15)
US 2002/034914 A1 (DE LEON SERGIO DIAZ [US] ET AL) 21 March 2002 (2002-03-21)

US 2004/127875A all disclose topsheets with protrusions and liquid channels therebetween. US 64 36082 for example discloses a corrugated sanitary napkin with peaks and ridges in a longitudinal direction, as well as some slightly weakened surface strips, formed transversely on the peaks, to assist in bending.

The invention is a topsheet as defined in claim 1.

In drawings which illustrate embodiments of the invention,
Fig. 1 is a fragmentary perspective view of the first preferred embodiment of a topsheet according to this invention;
Fig. 2 is a fragmentary sectional view of the first preferred embodiment;
Fig. 3 is a fragmentary perspective view of the second preferred embodiment of a topsheet according to this invention;
Fig. 4 is a fragmentary sectional view of the second preferred embodiment;
Fig. 5 is a fragmentary perspective view of the third preferred embodiment of a topsheet according to this invention;
Fig. 6 is a fragmentary sectional view of the third preferred embodiment;
Fig. 7 is a fragmentary perspective view of the fourth preferred embodiment of a topsheet according to this invention;
Fig. 8 is a fragmentary sectional view of the fourth preferred embodiment; and
Fig. 9 is a fragmentary sectional view of the fifth preferred embodiment of a topsheet according to this invention.

Before the present invention is described in greater detail with reference to the accompanying preferred embodiments, it should be noted herein that like elements are denoted by the same reference numerals throughout the disclosure.

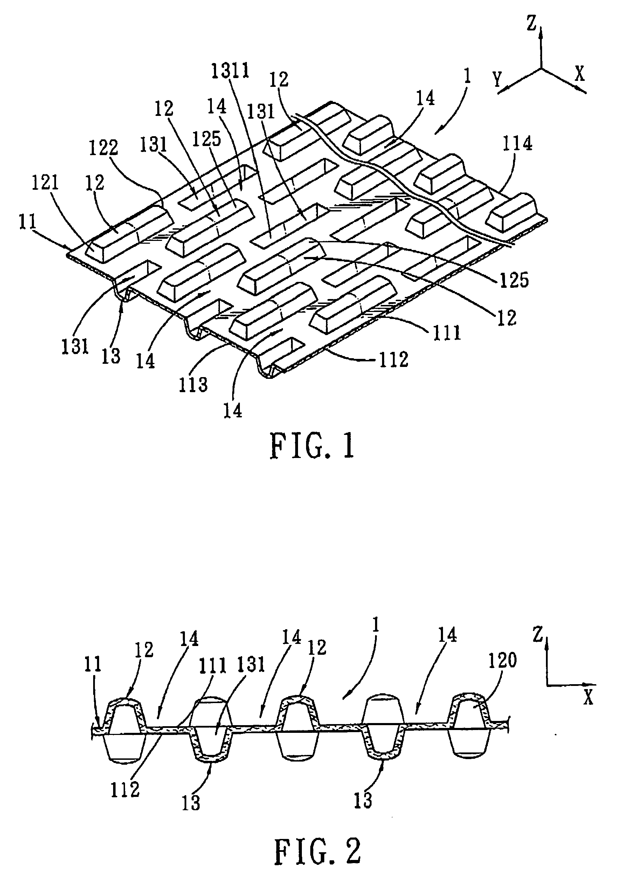

Figs. 1 and 2 illustrate the first preferred embodiment of a topsheet for an absorbent article, such as a sanitary napkin, an incontinence pad, a diaper, and the like, according to this invention. The topsheet includes a flexible sheet body 1 having a flat liquid pervious base wall 11 with first and second surfaces 111, 112 that are opposite to each other in a normal direction (Z) relative to the sheet body 1. The base wall 11 has front and rear edges 113, 114 that are opposite to each other in a longitudinal direction (Y) that is substantially perpendicular to the normal direction (Z).

The sheet body 1 further has a plurality of strip-like first protrusions 12 that are spaced apart from each other. The first protrusions 12 protrude from the first surface 111 of the base wall 11 in the normal direction (Z), and are distributed along the longitudinal direction (Y), as well as along a widthwise direction (X) which is transverse to the longitudinal direction (Y).

In this embodiment, the sheet body 1 is made from a non-woven fabric that is formed with a plurality of open pores for passage of a liquid (not shown) therethrough in the normal direction (Z). It is noted that formation of the non-woven fabric can be conducted in a conventional manner, such as by entangling fibers together using simple mechanical techniques that do not involve orienting fibers in a specific direction.

Each of the first protrusions 12 has front and rear ends 121, 122 that face toward the front and rear edges 113, 114 of the base wall 11, respectively, and extends lengthwise from the rear end 121 to the front end 122. The first protrusions 12 are arranged in such a manner to form a plurality of strip-like open fluid channels 14 thereamong. The fluid channels 14 extend from the front edge 113 to the rear edge 114 of the base wall 11, and have dimensions in the normal and widthwise directions (Z, X) sufficient to provide capillary action, thereby promoting spreading of a liquid body (not shown) on the first surface 111 of the base wall 11 in the longitudinal direction (Y).

In this embodiment, each of the first protrusions 12 is hollow and defines a recess that opens at the second surface 112 of the base wall 11.

The sheet body 1 further has a plurality of strip-like second protrusions 13 that protrude from the second surface 112 of the base wall 11, and that are distributed along the longitudinal direction (Y), as well as along the widthwise direction (X). Each of the second protrusions 13 is hollow, and defines a strip-like trough 131 that opens at the first surface 111 of the base wall 11 and that extends lengthwise in the longitudinal direction (Y). The troughs 131 defined by the second protrusions 13 are alternately disposed with the first protrusions 12. Each of the troughs 131 is partially aligned in the widthwise direction (X) with at least one of the first protrusions L2. That is, each of the troughs 131 has a section 1311 that is aligned in the widthwise direction (X) with a portion 125 of said at least one of the first protrusions 12. The troughs 131 have dimensions in the normal and widthwise directions (Z, X) sufficient to provide capillary action, thereby enhancing spreading of the liquid body in the longitudinal direction (Y).

In this embodiment, each of the first protrusions 12 extends lengthwise in the longitudinal direction (Y) so that each of the fluid channels 14 also extends in the longitudinal direction (Y). In addition, each of the fluid channels 14 is in fluid communication with an adjacent one of the fluid channels 14 so as to promote spreading cf the liquid body also in the widthwise direction (X).

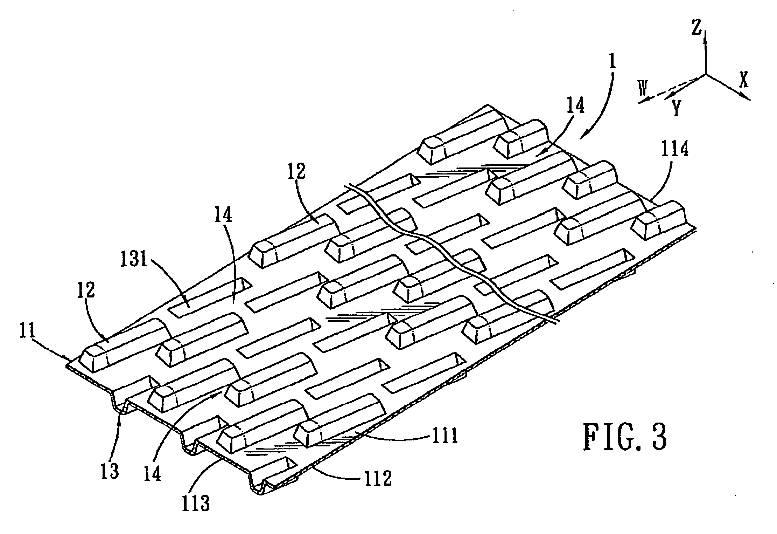

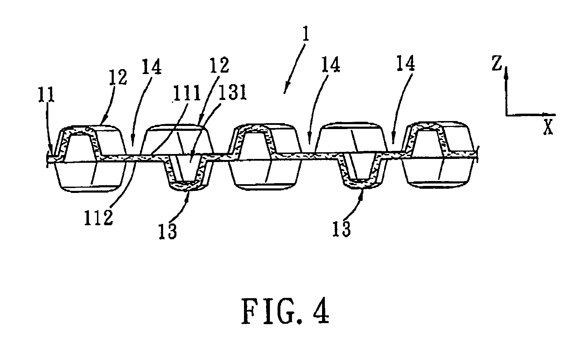

Figs. 3 and 4 illustrate the second preferred embodiment or the topsheet according to this invention. The second preferred embodiment differs from the previous embodiment in that each of the first protrusions 12 extends lengthwise in a direction (W) that is slightly inclined relative to the longitudinal direction (Y). In some embodiments, each of the second protrusions 13 also extends lengthwise in the direction (W).

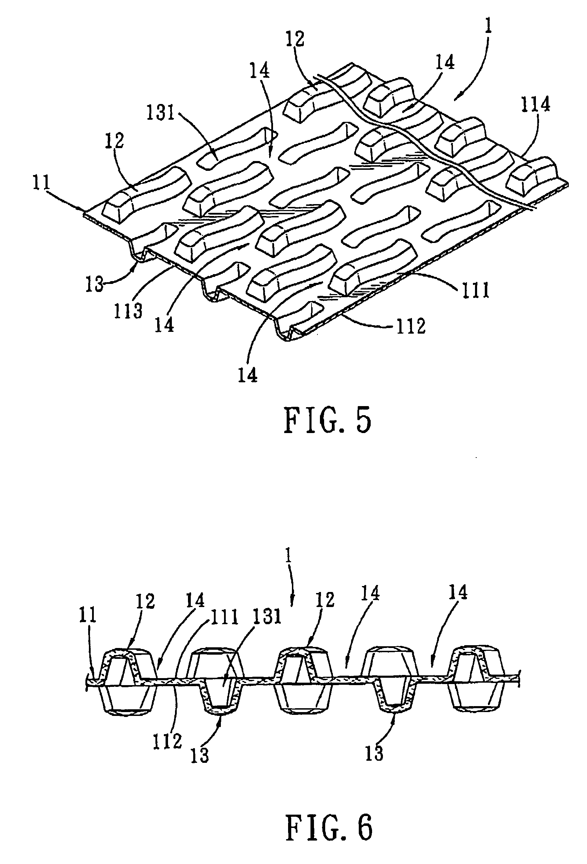

Figs. 5 and 6 illustrate the third preferred embodiment of the topsheet according to this invention. The third preferred embodiment differs from the first embodiment in that each of the first protrusions 12 is curvy in shape. That is, in the third preferred embodiments, each of the first protrusions 12 extends lengthwise in a slightly meandering manner. In some embodiments, each of the second protrusions 13 is also curvy in shape.

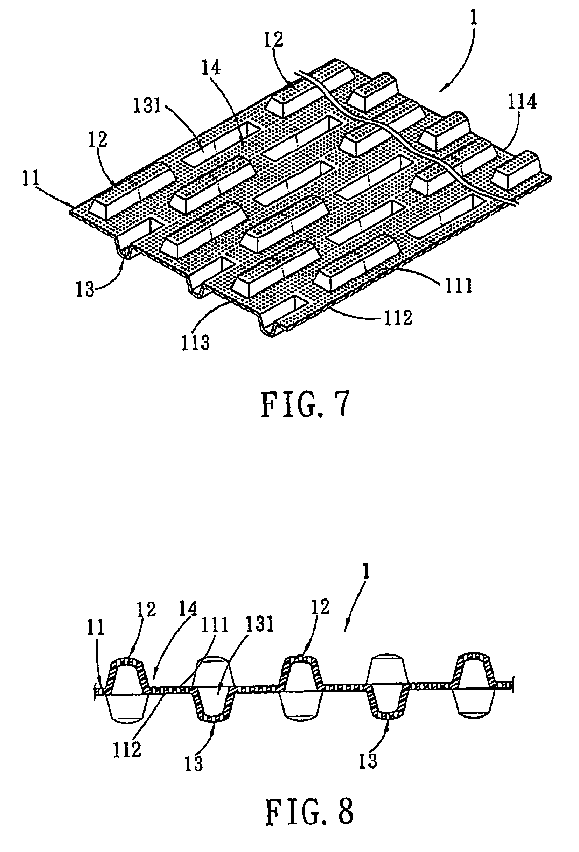

Figs. 7 and 8 illustrate the fourth preferred embodiment of the topsheet according to this invention. The fourth preferred embodiment differs from the first embodiment in that the sheet body 1 is made from a perforated plastic film which is formed with apertures for passage of liquid therethrough in the normal direction (Z). It is noted that in other preferred embodiments, the apertures can be formed only in the base wall 11 of the sheet body 1, and not in the first and second protrusions 12, 13 so that the first and second protrusions 12, 13 are liquid impervious.

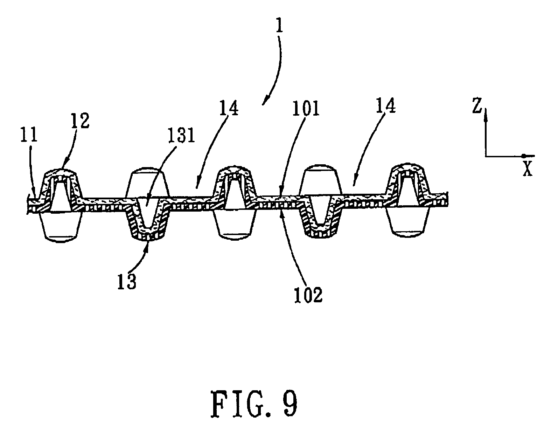

Fig. 9 illustrates the fifth preferred embodiment of the topsheet according to this invention. The fifth preferred embodiment differs from the first embodiment in that the sheet body 1 is made from a composite material having a bi-layer structure that has a non-woven layer 101 and a perforated plastic film layer 102 bonded to the non-woven layer 101.

With the inclusion of the first protrusions 12 and the troughs 131 in the sheet body 1 of the topsheet of this invention, a liquid body on the first surface 111 of the base wall 11 can rapidly spread in the longitudinal direction (Y), thereby considerably increasing the wet area of the base wall 11 over which liquid passes through both the base wall 11 as well as through walls that define the troughs 131. Ultimately, such a configuration of the present invention promotes transport of liquid into the absorbent article.

## Claims

1. A topsheet for an absorbent article, comprising:
a flexible liquid pervious sheet body (1) having a flat liquid pervious base wall (11) with first and second surfaces (111, 112) that are opposite to each other in a normal direction (Z) relative to said sheet body (1), said base wall (11) having front and rear edges (113, 114) that are opposite to each other in a longitudinal direction (Y), said sheet body (1) further having a plurality of strip-like first protrusions (12) that are spaced apart from each other, that protrude in the normal direction (Z) from said first surface (111) of said base wall (11), and that are distributed along the longitudinal direction (Y) as well as a widthwise direction (X) which is transverse to the longitudinal direction (Y), each of said first protrusions (12) having front and rear ends (121, 122) that face toward said front and rear edges (113, 114) of said base wall (11), respectively, and extending lengthwise from said rear end (122) to said front end (121);
wherein said first protrusions (12) are arranged in such a manner to form a plurality of strip-like open fluid channels (14) there among, said fluid channels (14) extending from said front edge (113) to said rear edge (114) of said base wall (11) and having dimensions in the normal and widthwise directions (Z, X) sufficient to provide capillary action, thereby prompting spreading of a liquid body on said first surface (111) of said base wall (11), each of the fluid channels (14) being in fluid communication with an adjacent one of the fluid channels (14) so as to promote spreading of the liquid body also in the widthwise direction (X);
said sheet body (1) further having a plurality of strip-like second protrusions (13) that protrude in the normal direction (Z) from said second surface (112) of said base wall (11), and that are distributed along the longitudinal direction (Y) as well as the widthwise direction (X), each of said second protrusions (13) being hollow and defining a strip-like trough (131) that opens at said first surfaces (111) of said base wall (11), said troughs (131) defined by said second protrusions (13) being alternately disposed with said first protrusions (12) along the longitudinal direction, the troughs (131) having dimensions in the normal and widthwise directions (Z,X) sufficient to provide capillary action, thereby enhancing spreading of the liquid body in the longitudinal direction (Y).

2. The topsheet of claim 1, in which each of the troughs (131) is partially aligned in the widthwise direction (X) with at least one of the first protrusions (12), such that each of the troughs (131) has a section (1311) that is aligned in the widthwise direction (X) with a portion (125) of said at least one of the first protrusions (12).

3. The topsheet of Claim 1, wherein each of said first protrusions (12) is hollow.

4. The topsheet of Claim 1, wherein each of said first protrusions (12) extends lengthwise in the longitudinal direction (Y) so that each of said fluid channels (14) extends in the longitudinal direction (Y).

5. The topsheet of Claim 1, wherein each of said first protrusions (12) extends lengthwise in a direction (W) inclined relative to the longitudinal direction (Y).

6. The topsheet of claim 1, wherein each of said strip-like first protrusions (12) is curvy in shape, extending lengthwise in a meandering manner.

7. The topsheet of Claim 1, wherein said sheet body (1) is made from a non-woven fabric.

8. The topsheet of Claim 1, wherein said sheet body (1) is made from a perforated plastic film having apertures for passage of liquid therethrough in the normal direction (Z).

9. The topsheet of Claim 1, wherein said sheet body (1) is made from a composite material having a bi-layer structure that has a non-woven layer (101) and a perforated plastic film layer (102) bonded to said non-woven layer (101).

10. The topsheet of claim B, wherein the apertures are formed only in the base wall (11) of the sheet body (1), and not in the first and second protrusions (12, 13), so that the first and second protrusions (12, 13) are Liquid impervious.

## Patentansprüche

1. Abdeckung für einen absorbierenden Gegenstand umfassend:
einen flexiblen flüssigkeitsdurchlässigen Abdeckungskörper (1) mit einer flachen flüssigkeitsdurchlässigen Basiswand (11) mit ersten und zweiten Flächen (111, 112), die einander in einer normalen Richtung (Z) relativ zum Abdeckungskörper (1) gegenüberliegen, wobei die Basiswand (11) vordere und hintere Ränder (113, 114) aufweist, die einander in Längsrichtung (Y) gegenüberliegen, wobei der Abdeckungskörper (1) ferner mehrere streifenartige erste vorstehende Teile (12) aufweist, die in Abständen voneinander angeordnet sind und die in der normalen Richtung (Z) von der ersten Fläche (111) der Basiswand (11) hervorstehen und die entlang der Längsrichtung (Y) sowie in Breitenrichtung (X), die quer zur Längsrichtung (Y) ist, verteilt sind, wobei jedes der ersten vorstehenden Teile (12) vordere und hintere Enden (121, 122) aufweist, die den vorderen und hinteren Kanten (113, 114) der Basiswand (11) gegenüberliegen und sich der Länge nach vom hinteren Ende (122) zum vorderen Ende (121) erstrecken;
wobei die ersten vorstehenden Teile (12) derart angeordnet sind, dass sie mehrere streifenartige offene Flüssigkeitskanäle (14) dazwischen bilden, wobei sich die Flüssigkeitskanäle (14) von der vorderen Kante (113) zur hinteren Kante (114) der Basiswand (11) erstrecken und Abmessungen in der normalen Richtung und der Breitenrichtung (Z, X) aufweisen, die ausreichend sind, um eine kapillare Wirkung bereitzustellen und dabei die Ausbreitung einer Flüssigkeit auf der ersten Fläche (111) der Basiswand (11) unterstützen, wobei jeder Flüssigkeitskanal (14) über die Flüssigkeit in Verbindung mit den angrenzenden Flüssigkeitskanälen (14) steht, um die Ausbreitung der Flüssigkeit auch in Breitenrichtung (X) zu unterstützen;
wobei der Abdeckungskörper (1) ferner mehrere streifenartige zweite vorstehende Teile (13) aufweist, die in der normalen Richtung (Z) von der zweiten Fläche (112) der Basiswand (11) hervorstehen und die entlang der Längsrichtung (Y) sowie der Breitenrichtung (X) verteilt sind, wobei die zweiten vorstehenden Teile (13) hohl sind und einen streifenartigen Trog (131) definieren, der an der ersten Fläche (111) der Basiswand (11) offen ist, wobei die Tröge (131) von den zweiten vorstehenden Teilen (13) definiert sind, die abwechselnd mit den ersten vorstehenden Teilen (12) entlang der Längsrichtung angeordnet sind, und die Tröge (131) Abmessungen in der normalen Richtung und der Breitenrichtung (Z, X) aufweisen, die ausreichend sind, eine kapillare Wirkung bereitzustellen und damit die Ausbreitung der Flüssigkeit in Längsrichtung (Y) zu unterstützen.

2. Abdeckung nach Anspruch 1, wobei jeder der Tröge (131) teilweise in Breitenrichtung (X) mit mindestens einem der ersten vorstehenden Teile (12) derart ausgerichtet ist, dass jeder der Tröge (131) einen Bereich (1311) aufweist, der in Breitenrichtung (X) mit einem Bereich (125) von mindestens einem der ersten vorstehenden Teile (12) ausgerichtet ist.

3. Abdeckung nach Anspruch 1, wobei jedes der ersten vorstehenden Teile (12) hohl ist.

4. Abdeckung nach Anspruch 1, wobei jedes der ersten vorstehenden Teile (12) sich der Länge nach in Längsrichtung (Y) erstreckt, derart, dass sich jeder der Flüssigkeitskanäle (14) in Längsrichtung (Y) erstreckt.

5. Abdeckung nach Anspruch 1, wobei sich jedes der ersten vorstehenden Teile (12) in Längsrichtung (W), die schräg zur Längsrichtung (Y) ist, erstreckt.

6. Abdeckung nach Anspruch 1, wobei jedes der streifenartigen ersten vorstehenden Teile (12) bogenförmig ausgebildet ist und sich gewunden der Länge nach erstreckt.

7. Abdeckung nach Anspruch 1, wobei der Abdeckungskörper (1) aus Faserstoff hergestellt ist.

8. Abdeckung nach Anspruch 1, wobei der Abdeckungskörper (1) aus einer perforierten Kunststofffolie mit Öffnungen zum Durchtritt von Flüssigkeit in normaler Richtung (Z) hergestellt ist.

9. Abdeckung nach Anspruch 1, wobei der Abdeckungskörper (1) aus einem Verbundmaterial hergestellt ist und eine zweilagige Struktur, die eine Faserschicht (101) und eine perforierte Kunststofffilmschicht (102), die auf die Faserschicht (101) geklebt ist, aufweist.

10. Abdeckung nach Anspruch 8, wobei die Öffnungen nur in der Basiswand (11) des Abdeckungskörpers (1) und nicht in den ersten und zweiten vorstehenden Teilen (12, 131) ausgebildet sind, derart, dass die ersten und zweiten vorstehenden Teile (12, 13) flüssigkeitsundurchlässig sind.

## Revendications

1. Une feuille de dessus pour un article absorbant, comprenant :
un corps de feuille souple perméable aux liquides (1) ayant une paroi de base plate perméable aux liquides (11) avec une première et une deuxième surfaces (111, 112) qui sont placées en face l'une de l'autre dans un sens perpendiculaire (Z) par rapport au dit corps de feuille (1), la dite paroi de base (11) ayant des bords avant et arrière (113, 114) qui sont placés l'un en face de l'autre dans un sens longitudinal (Y), le dit corps de feuille (1) ayant en outre une pluralité de premières saillies (12) ressemblant à des bandes, qui sont espacées les unes des autres, qui dépassent dans le sens perpendiculaire (Z) de la dite première surface (111) de la dite paroi de base (11), et qui sont réparties le long du sens longitudinal (Y), de même que dans le sens de la largeur (X) qui est transversal par rapport au sens longitudinal (Y), chacune des dites premières saillies (12) ayant des extrémités avant et arrière (121, 122) qui sont tournées vers les dits bords avant et arrière (1113, 114) de la dite paroi de base (11), respectivement, et s'étendant longitudinalement entre la dite extrémité arrière (122) et la dite extrémité avant (121) ;
où les dites premières saillies (12) sont placées de sorte à former entre elles une pluralité de canaux à fluide ouverts (14) ressemblant à des bandes, les dits canaux à fluide (14) s'étendant entre le dit bord avant (113) et le dit bord arrière (114) de la dite paroi de base (11) et ayant des dimensions, dans les sens perpendiculaire et de la largeur (Z, X), suffisantes pour assurer une action capillaire, en favorisant ainsi la répartition d'un corps liquide sur la dite première surface (111) de la dite paroi de base (11), chacun des canaux à fluide (14) étant en communication par les fluides avec un l'un des canaux à fluide (14), adjacent, de façon à favoriser la répartition du corps liquide dans le sens de la largeur (X) également ;
le dit corps de feuille (1) ayant par ailleurs une pluralité de deuxièmes saillies (13) ressemblant à des bandes qui dépassent dans le sens perpendiculaire (Z) de la dite deuxième surface (112) de la dite paroi de base (11), et qui sont réparties le long du sens longitudinal (Y), de même que dans le sens de la largeur (X), chacune des dites deuxièmes saillies (13) étant creuse et définissant un auget (131) ressemblant à une bande qui s'ouvre au niveau de la dite première surface (111) de la dite paroi de base (11), les dits augets (131) définis par les dites deuxièmes saillies (13) étant alternativement disposés avec les dites premières saillies (12) le long du sens longitudinal, les augets (131) ayant des dimensions, dans les sens perpendiculaire et de la largeur (Z, X), suffisantes pour assurer une action capillaire, en augmentant ainsi la répartition du corps liquide dans le sens longitudinal (Y).

2. La feuille de dessus de la revendication 1, dans laquelle chacun des augets (131) est partiellement aligné dans le sens de la largeur (X) sur au moins l'une des premières saillies (12), de sorte que chacun des augets (131) présente une section (1311) qui est alignée dans le sens de la largeur (X) sur une partie (121) d'au moins l'une des dites premières saillies (12).

3. La feuille de dessus de la revendication 1, où chacune des dites premières saillies (12) est creuse.

4. La feuille de dessus de la revendication 1, où chacune des dites premières saillies (12) s'étend longitudinalement dans le sens longitudinal (Y), de sorte que chacun des dits canaux à fluide (14) s'étende dans le sens longitudinal (Y).

5. La feuille de dessus de la revendication 1, où chacune des dites premières saillies (12) s'étend longitudinalement dans un sens incliné par rapport au sens longitudinal (Y).

6. La feuille de dessus de la revendication 1, où chacune des dites premières saillies (12) ressemblant à des bandes est de forme incurvée et s'étend longitudinalement de façon sinueuse.

7. La feuille de dessus de la revendication 1, où le dit corps de feuille (1) est constitué d'un tissu non tissé.

8. La feuille de dessus de la revendication 1, où le dit corps de feuille (1) est constitué un film en plastique perforé ayant des ouvertures pour permettre au liquide de traverser, dans le sens perpendiculaire (Z).

9. La feuille de dessus de la revendication 1, où le dit corps de feuille (1) est constitué d'un matériau composite ayant une structure en bi-couche qui comporte une couche non tissée (101) et une couche en film en plastique perforé (102) collée sur la dite couche non tissée (101).

10. La feuille de dessus de la revendication 8, où les ouvertures ne sont formées que dans la paroi de base (11) du corps de feuille (1), et non pas dans les premières et deuxièmes saillies (12, 13), de sorte que les premières et deuxièmes saillies (12, 13) soient imperméables au liquide.
